# EUROPEAN PATENT SPECIFICATION

(11) **EP 3 145 472 B1**
(45) Date of publication and mention of the grant of the patent: **18.04.2018**
(21) Application number: 15732048.2
(22) Date of filing: 18.05.2015
(51) Int. Cl.: A61K 8/04, A45D 40/26, A45D 34/04, B05B 7/00

(54) **DEVICE FOR PACKAGING AND APPLYING A COSMETIC OR DERMATOLOGICAL PRODUCT**
VORRICHTUNG ZUM VERPACKEN UND AUFTRAGEN EINES KOSMETISCHEN ODER DERMATOLOGISCHEN PRODUKTS
DISPOSITIF DE CONDITIONNEMENT ET APPLICATION D'UN PRODUIT COSMÉTIQUE OU DERMATOLOGIQUE

(30) Priority: 20.05.2014 FR 1454526
(43) Date of publication of application: 29.03.2017
(73) Proprietor: L'OREAL, 75008 Paris (FR)
(72) Inventor: CAULIER, Eric, F-60420 Ferrieres (FR); ROUDAUT, Etienne, F-93588 Saint Ouen (FR)
(74) Representative: Nony
(86) International application number: PCT/IB2015/053648
(87) International publication number: WO 2015/177706

(56) References cited:
- EP-A2- 0 715 820
- FR-A1- 2 629 695
- FR-A1- 2 850 545

## Description

The present invention relates to a device for packaging and applying a cosmetic or dermatological product, for example for application to the skin, and more particularly the devices comprising a grid for applying the product.

### Background

The patent US 5 725 155 proposes using, at the outlet of a foam aerosol, a finemesh screen made of metal or plastic. This screen is positioned in an end piece defining, upstream of the screen, a single duct of circular cross section.

Patent US 6 309 128 teaches the use, at the outlet of a reservoir containing a product to be applied, of a flexible application element forming an application surface for cosmetic product, comprising a plurality of passages allowing product to be distributed towards the application surface. The application element is supplied with product via a single duct of circular cross section.

Application EP 0 715 820 relates to an end piece for applying a product and a distributor equipped with this end piece. The end piece comprises an application surface provided with at least one distribution orifice. The application surface can be defined by a grid formed with an end piece which caps the neck of a receptacle containing the product, of circular cross section.

Patent US 7 147 395 describes a device for applying a product, comprising, at the outlet, a metal mesh application member. This application member is supplied via a single opening.

Application EP 2 245 959 describes a distributor for a product by means of which it is possible to make the product foam, comprising a distribution and application element made of a material of the lattice, mesh, gauze or woven type, having a plurality of egress channels. The distribution element is spread in the form of a dome over spacer elements of an element forming a base, allowing product from the receptacle to be distributed at the distribution element. The spacer elements make it possible to space the distribution element from the element forming a base, so as to form channels between the two. A single central passage passes through the base. One drawback of this distributor is that in the case of distributing a viscous product, it is difficult for the product to spread out beneath the distribution element, such that it tends to remain confined at the centre of the element as it exits. Such a distributor is thus limited to the distribution of low-viscosity product.

There exists a need for a distribution and application device by means of which it is possible to further improve the application of cosmetic or dermatological products to human keratinous materials, in particular the skin, in order in particular to improve the texture of the applied product.

### Summary

The invention aims to meet this need and it achieves this by virtue of a device for packaging and applying a cosmetic or dermatological product, comprising:
- a receptacle containing said cosmetic or dermatological product,
- an applicator head which is supplied with product by the receptacle, the applicator head comprising:
   ∘ a flexible grid defining an application surface for applying the product onto a surface to be treated,
   ∘ a partitioned support element for the grid, against which the grid can rest at least during application, having at least one partition between two regions where the product can pass through the support element.

A "partitioned support element" refers to a support element for the grid, having an outlet for the product comprising a plurality of open regions for distributing the product towards the grid, which regions are separated by one or more partitions; the distribution regions may be discontinuous and form a plurality of openings allowing the axial distribution of the product to the grid. As a variant, the distribution regions are not discontinuous, opening onto one another and forming a single opening allowing the axial distribution of the product to the grid, with the grid resting on the partitions. The opening preferably has a circular overall shape on its radially outermost contour, and the partitions are oriented radially in the opening.

Preferably, the opening or openings open axially, that is to say that the product can pass through them to supply the grid, flowing in line with the applicator head.

The support element makes it possible to distribute, through its opening or openings, the product to the grid and to allow the product to pass trough part of the surface of the grid while supporting the latter, in particular during application. As the cosmetic product passes through the grid, it is aerated and takes on a more foamy appearance, which can make it visually more agreeable. The grid also improves the texture of the product and can make application more precise and comfortable by making it easier to spread the product.

The support element also makes it possible to obtain a pattern effect on the product as the latter exits the applicator head. The product exits the grid through those parts which overlap the opening or openings, and the regions of the grid overlapping the solid parts of the support element, that is to say the partitions, tend not to have product passing through them. The product thus takes on, upon exiting the grid, a pattern which depends on the shape of the support element.

Obtaining a pattern effect has two advantages. First, it provides the mass of product present on the grid before application with a pleasant aesthetic aspect. Moreover, it makes it possible to reduce the amount of product appearing on the grid during distribution of the product, as the product accumulates essentially only at the locations of the opening or openings of the support element. It is easier to avoid the risk of distributing an excess of product on the grid, which permits a more precise application and saves product.

Preferably, the cosmetic product has a semi-solid galenical form, having for example the texture of a cream, a gel or a paste. The product may have a viscosity at ambient temperature of between 5 Pa.s and 20 Pa.s, preferably between 7 Pa.s and 19 Pa.s, measured at 25°C with a Rheomat 180 viscometer equipped with a n° 4 bob, the measurement being taken after 10 min of rotation of the bob at a shear rate of 200 min⁻¹. Preferably, the product is sufficiently viscous to tension the grid during distribution, when the user presses on the wall of the receptacle or otherwise subjects the product contained in the receptacle to pressure. The viscosity of the product is preferably high enough that the product, after passing through the grid, does not entirely recover its cohesion by re-adopting a smooth surface aspect. In other words, the passage through the grid can give the product present on the grid, after passing through, a surface state with a micro-relief which corresponds to the mesh of the grid.

Preferably, the grid is formed from a textile.

A "textile" refers to a flexible material which can be divided into natural or artificial fibres or threads. The textile can be a woven, that is to say a flexible material consisting of interlaced threads, or a non-woven, that is to say a material whose fibres are held together in a random fashion. The textile can be a part assembled with the remainder of the device. As a variant, at least part of the device can be overmoulded onto the textile.

The grid can furthermore be an injected or extruded net or a micro-perforated film.

The textile can be perforated or non-perforated, depending on the size of the openings which are to be obtained within the grid for the product to pass through.

The grid, in particular the textile, is preferably made of a thermoplastic material, in particular polyamide 6.6, polyester or PP.

The grid, in particular the textile, can comprise a plurality of threads of diameter between 2 µm and 1000 µm, preferably between 50 µm and 250 µm, more preferably between 60 µm and 150 µm.

The textile of the grid preferably comprises between 20 threads/cm and 70 threads/cm, preferably between 28 threads/cm and 35 threads/cm.

The grid is preferably formed from a woven or a net with a mesh having openings of cross section between 0.01 mm² and 1 mm², preferably between 0.02 mm² and 0.1 mm², more preferably between 0.03 mm² and 0.05 mm².

The thickness of the grid is preferably less than 1 mm, preferably less than 0.5 mm, more preferably less than 0.3 mm.

The threads of the textile or of the net are preferably round in cross section. As a variant, the threads of the textile or of the net may have any cross section, in particular polygonal, or comprising a flat portion or elongate.

Preferably, the grid, in particular the textile or the net, is inextensible. This allows the grid to not deform excessively when subjected to the pressure of the product, and to retain the pattern effect during distribution. As a variant, the grid may be at least partially extensible.

Preferably, the grid and in particular the threads of the textile or of the net are not flocked. It is possible, by virtue of the invention, to obtain pleasant contact with the skin in the absence of a flocking coating.

The applicator head is preferably domed towards the surface to be treated. Preferably, the grid is domed towards the surface to be treated. The grid, which covers the support element, matches the general shape of the latter and has an aspect which is domed outwards, which facilitates the application of the product. Preferably, the grid is domed by a height between 0.1 and 6 mm, preferably between 0.2 and 1 mm, this height being measured in line with the axis of the head, between the periphery of the grid and the summit of the latter, on its apparent external face.

Preferably, the grid comes into direct contact with the support element, and in particular its partitions. The support element supports the grid, preventing, in particular, the latter from collapsing during application or from becoming domed towards the interior of the applicator head.

The product can be applied directly, or the user can remove, in particular using a finger, the product from the surface of the grid in order to apply it subsequently.

The applicator head can comprise two parts. It can comprise an insert which is attached, in particular snap-fitted, onto the receptacle, or which is forced into the receptacle, and a support part which bears the grid and is attached to the insert or the receptacle, in particular snap-fitted onto the insert or the receptacle. The support part and the insert or the receptacle may comprise cooperating anti-rotation reliefs, in particular cooperating splines.

The support element may comprise a plurality of partitions, the partitions being a central region and connection bridges connected to the central region, the connection bridges defining, between them, open-worked portions forming a plurality of openings. The central region may be open-worked.

The grid may come into contact with the central region and with connection bridges. As a variant, the connection bridges may be arranged back from the central region and not serve as supports for the grid, the latter coming to press against the central region.

The support element can form a pattern on the product which passes through the grid by virtue of the arrangement and the shape of the open-worked portions.

Preferably, the support element is in the shape of a dome which is convex outwards.

Preferably, the support element comprises between 2 and 20 connection bridges, preferably between 3 and 20 connection bridges, more preferably between 6 and 20 connection bridges, for example between 6 and 12 connection bridges, preferably between 6 and 10 connection bridges.

The support element can be part of the support part and in particular can be moulded with the support part. The latter can be moulded in contact with the grid or with a ring overmoulded onto the grid. As mentioned above, the material of the support element can, if necessary, extend through the mesh of the grid in the event that it is overmoulded onto the latter to adhere to the grid.

The support element may comprise a plurality of partitions, the partitions being fins connected to a central region, the fins defining, between them, axial passages for the product. Preferably, the fins come into contact with the grid with their upper edge.

The support element is preferably arranged on the insert and comes into contact with the grid when the insert is assembled with the support part and makes it possible to support the grid so that it retains its domed shape. The upper edges of the fins can locally obscure the grid and form a pattern on the product which flows through the grid.

Preferably, the support element is moulded with the insert.

The fins are preferably secured to a jet break which connects them and make it possible in particular to connect the latter to the insert. Preferably, the fins project beyond the support part by a height between 0.1 and 1 mm, preferably between 0.2 and 0.5 mm.

The grid can adhere to the partitions of the support element, the latter being for example moulded on contact therewith, which can cause localized melting between the material of the grid and of the partitions. As a variant, the grid remains free with respect to the partitions, the grid being for example attached to the support part, after formation of the partitions.

When the support element is created with the support part, then preferably the partitions are moulded in contact with the grid. When the support element is created with the insert assembled with the support part, then the partitions are preferably moulded separately from the grid.

Preferably, the applicator head, in particular the insert, comprises a sealing skirt which engages in the receptacle.

The applicator head, in particular the insert, may comprise a jet break which makes it possible to improve the distribution of the product over the entire grid. The jet break may be arranged at the exit from the receptacle, in particular above the passage defined by the sealing skirt. The jet break may comprise a raised platform connected to the rest of the applicator head by at least one rigid element. As a variant, the jet break comprises a plurality of arches connected to one end of the rest of the applicator head and the other end extends as far as a point which is raised with respect to the passage defined by the sealing skirt.

Preferably, the jet break is created such that, when the product is distributed through the grid, the height of product which accumulates on the grid, at each axial passage for the product defined by the support element, is essentially the same. Thus, when the support element has a central opening and peripheral openings formed between connection bridges of the support element, the jet break is preferably created such that the height of product which accumulates at the centre of the grid is essentially of the same order as that which accumulates at the peripheral openings arranged around the central opening.

Preferably, the jet break is moulded with at least part of the applicator head, in particular the insert.

Preferably, the applicator head, in particular the insert, is attached to the receptacle, in particular secured by snap-fitting onto the receptacle or by being forced into the receptacle. The applicator head, in particular the insert, and the receptacle may comprise cooperating anti-rotation reliefs, in particular cooperating projecting elements and recesses.

The applicator head, in particular the support part, may comprise a portion overmoulded onto the grid, more particularly when the latter consists of a textile or a net.

As a variant, the applicator head, in particular the support part, may comprise a portion which is attached to a support ring, in particular overmoulded onto the support ring. The latter may itself be overmoulded onto the grid.

Preferably, the device comprises a cap by means of which the applicator head can be covered when the device is not in use. The cap makes it possible, when the device is not in use, to protect the grid and the product from the external environment.

The receptacle may have a variable internal volume, the supply of product coming from the receptacle to the applicator head being brought about by pressure exerted on a wall of the receptacle.

As a variant, the supply of product from the receptacle to the applicator head is brought about with the aid of a piston.

A further subject not of the invention is a method for producing an applicator head, comprising the steps of:
- integrating a pre-cut grid, preferably a textile, onto a support ring, and
- integrating the support ring and the flexible grid onto at least one portion of the applicator head.

The support ring and the flexible grid may be integrated by mounting, snap-fitting or overmoulding onto the portion of the applicator head.

The support ring can be overmoulded onto the flexible grid.

The material of the support ring can extend through the mesh of the grid, in particular when the latter is a woven or a net, which can improve the anchoring of the grid in the support ring. The latter and the grid may be made of different materials, the grid being preferably made of a material having a higher melting temperature. As a variant, the grid is assembled onto the ring. In particular, the ring may be in two parts, the first receiving the grid and the second being fixed over the grid, on the first part, so as to clamp the grid between the two parts.

The abovementioned portion of the applicator head can be overmoulded onto the support ring and the grid.

The invention may be better understood from reading the following detailed description of non-limiting implementation examples thereof and from examining the appended drawing, in which:
- Figure 1 represents a partial perspective view, in axial cross section, of a device according to the invention,
- Figure 2 is a partial view in axial cross section of a variant of a device according to the invention,
- Figure 3 shows a partial exploded view of the device from Figure 1,
- Figures 4 and 5 show the insert in isolation and in perspective,
- Figure 6 is a view in axial cross section of the insert from Figures 4 and 5,
- Figures 7 and 8 show, in isolation, a support part according to the invention,
- Figure 9 is a view in cross section of the support part from Figures 7 and 8,
- Figure 10 is a top view of a textile grid in accordance with one exemplary embodiment of the invention,
- Figure 11 is a view of the applicator head according to the invention, after the product has been distributed onto the grid,
- Figure 12 illustrates steps of a method for producing the applicator head,
- Figure 13 is a view in cross section of a variant embodiment of the invention, and
- Figure 14 shows an exploded view of the device from Figure 13, and
- Figure 15 is a top view of a support element variant.

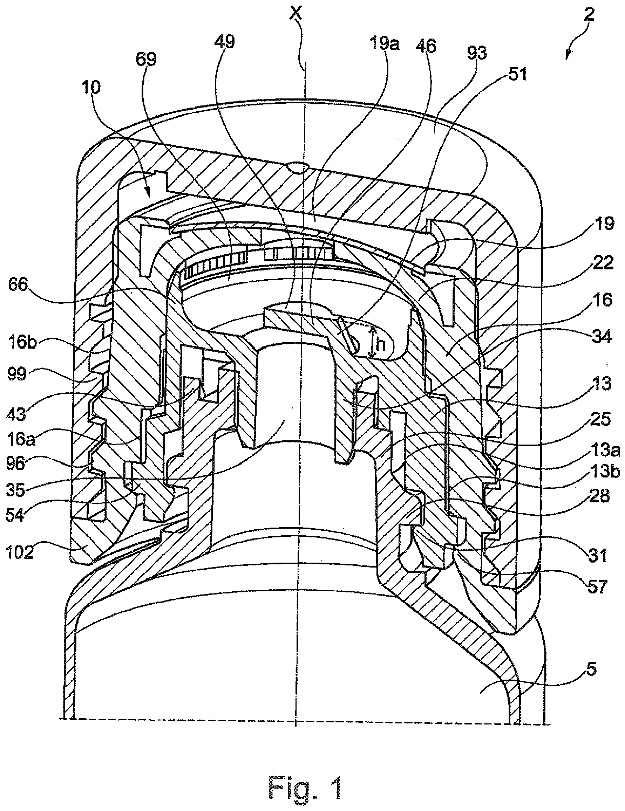

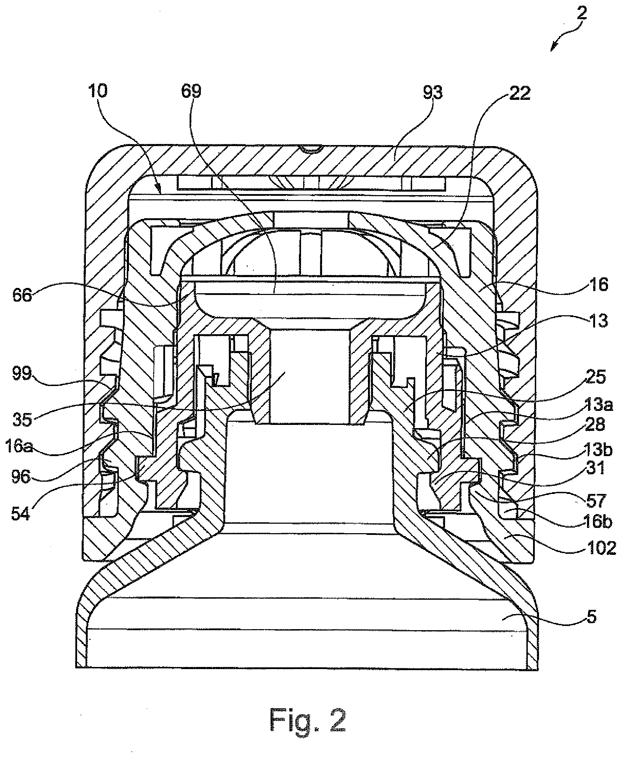

The packaging and application device 2 shown in Figures 1 and 2 comprises a receptacle 5 containing the product to be applied and an applicator head 10.

The latter comprises an insert 13 attached to the neck 25 of the receptacle 5 and a support part 16 attached to the insert 13 and comprising a grid 19 defining an application surface 19a, arranged on a support element 22 of the support part 16.

The receptacle 5 containing the product preferably has a variable internal volume. The applicator head 10 can be supplied with product from the receptacle 5 by means of pressure on the wall of the receptacle 5 and/or with the aid of a piston (or pump).

The receptacle 5 may be flexible or rigid and the product may be contained in a flexible bag, if relevant.

Preferably, the cosmetic product P has a semi-solid galenical form, having for example the texture of a cream, a gel or a paste. The product P may have a viscosity at ambient temperature of between 5 Pa.s and 20 Pa.s, preferably between 7 Pa.s and 19 Pa.s, measured at 25°C with a Rheomat 180 viscometer equipped with a n° 4 bob, the measurement being taken after 10 min of rotation of the bob at a shear rate of 200 min⁻¹. Preferably, the product is sufficiently viscous to stretch the grid as it is distributed.

It may be a foundation.

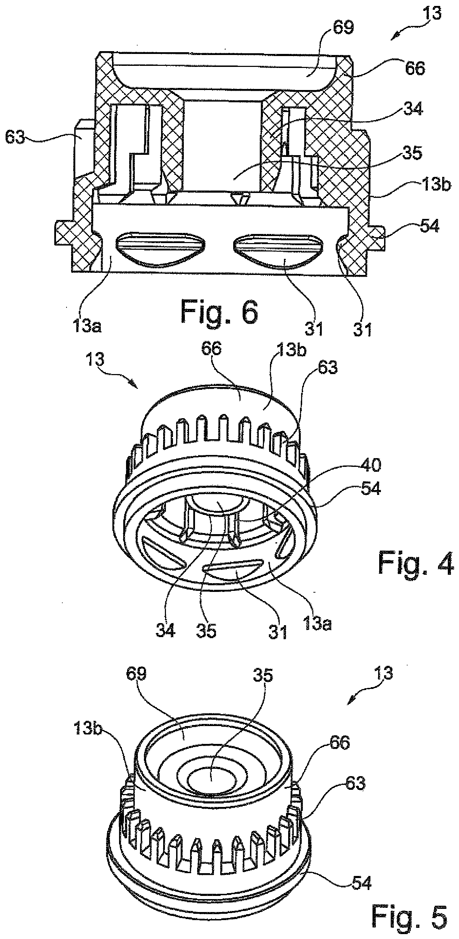

The neck 25 may comprise, as shown, a collar 28 onto which the insert 13, shown more particularly in Figures 4 to 6, is snap-fitted by means of first snap-fitting reliefs 31 which are spaced apart over the periphery of its internal face 13a.

The insert 13 preferably comprises a sealing skirt 34 which engages in the neck 25 of the receptacle 5 and forms a passage 35, to the outside, for the product contained in the receptacle 5.

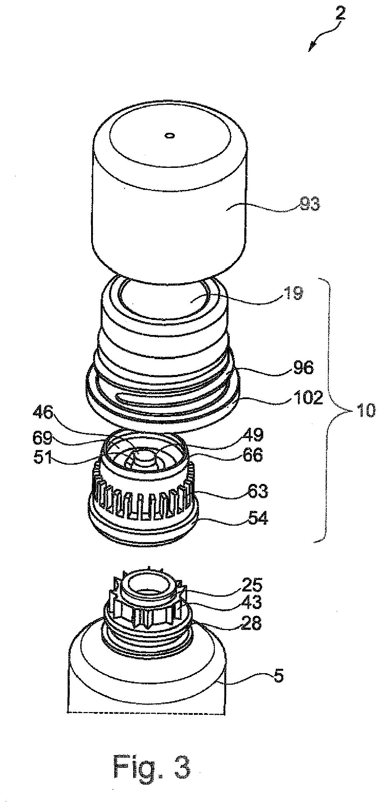

Preferably, the insert 13 and the receptacle 5 comprise corresponding anti-rotation reliefs, in particular splines. In the example in question, the insert 13 comprises recesses 40, shown in Figure 4, which are designed to receive the projecting elements 43 of the neck 25 of the receptacle 5, shown in Figure 3.

The insert 13 may comprise a jet break 46 at the outlet of the passage 35, formed by the sealing skirt 34. Preferably, the jet break 46 comprises a plate 49 arranged perpendicular to the axis X along which the product exits, such that the product leaving the receptacle 5 is diverted and spreads out laterally.

The plate 49 may have a diameter which is essentially equal to that of the passage 35, and be raised with respect to the outlet of the passage 35. Preferably, the jet break 46 is arranged at a height h between 0.5 mm and 5 mm from the outlet of the passage 35. The jet break 46 is connected to the insert 13 by a material bridge 51. The jet break 46 and the material bridge 51 are preferably moulded in one piece with the insert 13.

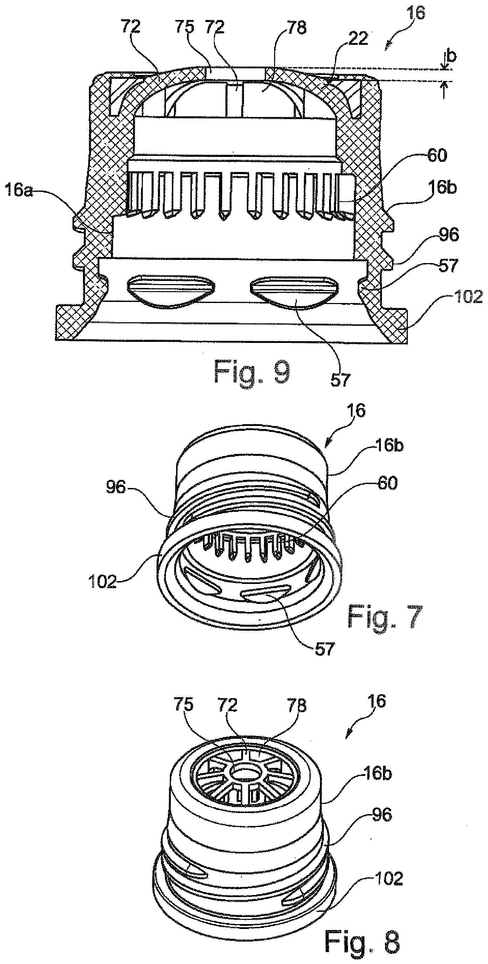

The insert 13 may comprise, on its external face 13b, a bead 54 onto which the support part 16, shown in detail in Figures 7 to 9, is snap-fitted by means of second snap-fitting reliefs 57 which are spaced apart over the periphery of its internal face 16a.

Preferably, the support part 16 and the insert 13 comprise corresponding anti-rotation reliefs, in particular splines. The support part 16 may comprise in particular first splines 60, illustrated in Figure 7, which are designed to receive second splines 63 of the external face 13b of the insert 13, shown in Figures 3 and 4.

The sealing between the insert 13 and the support part 16 may be obtained by means of an annular sealing skirt 66 of the insert 13, which comes to press against the internal face 16a of the support part 16.

The insert 13 and the support part 16 define, between them, a cavity 69 into which opens the passage 35 and which is designed to fill with product in order to supply the grid 19 with product.

As shown in Figure 8, the support element 22 is preferably domed outwards and comprises partitions in the form of an annular central region 75 and connection bridges 72 connected to the annular central region 75. The connection bridges define, between them, discontinuous openings 78. The annular central region 75 defines a central opening.

The grid 19 is preferably in contact with the connection bridges 72 of the support member 22, against which it rests.

The grid 19 is supplied with product through openings 78. The grid 19 is preferably inextensible. However, this may not be the case.

The grid 19 can adhere to the connection bridges 72, the latter being moulded in contact therewith. As a variant, the grid 19 rests against the connection bridges 72 without being directly attached thereto locally by adhesive bonding or by melting material.

Preferably, as shown, the grid 19 has a circular contour. As a variant, the grid 19 has another contour, in particular polygonal or oblong.

Preferably, as shown, the grid is domed towards the surface to be treated because it bears on the support member.

The grid 19 is preferably made of a thermoplastic material, in particular polyamide 6.6, PA, PET or PP.

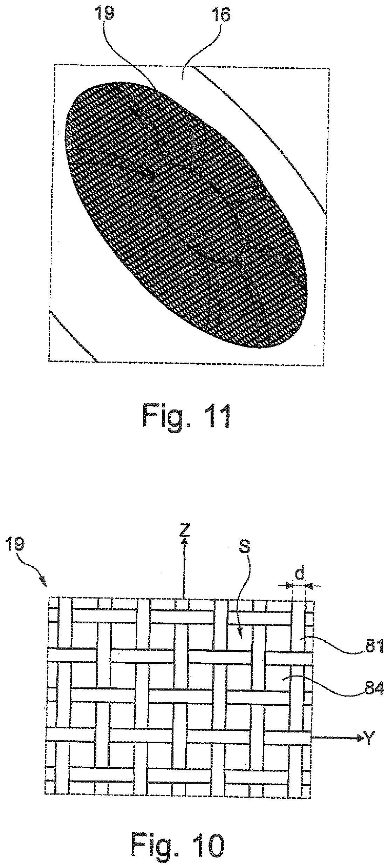

As shown in Figure 11, the grid may be a textile and the textile of the grid 19 is preferably a woven and comprises a plurality of threads 81 which are interlaced and arranged along two mutually perpendicular axes Y and Z, and which between them define mesh openings 84.

The textile of the grid 19 can comprise a plurality of threads 81 of diameter *d* between 2 µm and 1000 µm, preferably between 50 µm and 250 µm, more preferably between 60 µm and 150 µm.

The textile of the grid 19 preferably comprises between 20 threads/cm and 70 threads/cm, preferably between 28 threads/cm and 35 threads/cm.

The mesh openings 84 of the textile of the grid 19 preferably each have a cross section S between 0.01 mm² and 1 mm², preferably between 0.01 mm² and 0.1 mm², more preferably between 0.03 mm² and 0.05 mm².

The thickness of the grid 19 is preferably less than 1 mm, preferably less than 0.2 mm.

In variants which have not been shown, the grid 19 is formed from a microperforated film or from an extruded net; the grid 19 may also be formed from a perforated nonwoven.

During distribution of the product, the product passes, along the X axis of the applicator head 10, through the openings 78 of the support element 22 and comes into contact with the grid 19 so as to pass through it in line with the openings 78.

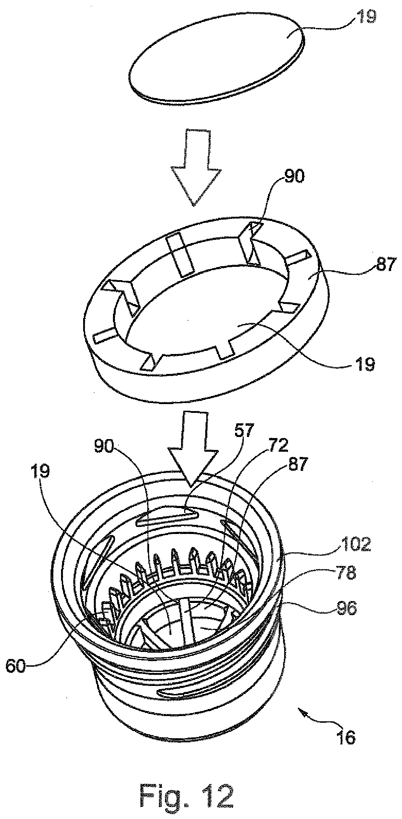

Preferably, as shown in Figure 12, the product is distributed onto the grid 19 only at those of its regions which overlap the openings 78, and thus reproduces the pattern defined by the openings 78.

During application onto the surface to be treated, the grid may be moved on the skin. As a variant, the user may remove the product directly from the grid so as to apply it to the skin, in particular with a finger.

The support part 16 may be configured so as to receive a cap 93. The latter covers the applicator head 10 so as to protect the grid 19 from the outside environment, in particular so as to prevent the product from drying out when exposed to the air. The device may comprise a seal which is positioned between the support part 16 and the cap 93 and which allows the cap 93 to be closed in a sealed manner.

Preferably, as shown, the support part 16 comprises, on its external face 16b, a thread 96 for engaging with a thread 99 of the cap 93. The cap 93 can abut against a collar 102 of the support part.

Preferably, the insert 13, the support part 16 and the cap 93 are made of a thermoplastic material, in particular polypropylene.

Preferably, the insert 13, the support part 16 and the cap 93 are injection-moulded.

As shown in Figure 10, the support part 16 may be manufactured by overmoulding a support ring 87 onto the grid 19, then overmoulding the remainder of the support part 16 onto the support ring 87.

Preferably, the grid 19 is domed outwards after overmoulding the support ring 87. The grid 19 is preferably domed by a height b, as shown in Figure 9, between 0 and 6 mm, preferably between 0.1 and 1 mm, preferably between 0.2 and 0.5 mm.

As a variant (not shown), the grid 19 may be assembled on the support ring 87. The support part may comprise two parts, between which is fixed the grid 19. Preferably, the first part receives the grid 19 and the second part covers the grid 19, which is positioned on the first part, and clamps it between the external surface of the first part and the internal surface of the second part, such that the grid 19 is held in position by being clamped between the two parts.

Preferably, the support ring 87 comprises notches 90 for receiving the connection bridges 72 of the support element 22. The latter can adhere to the grid 19, being for example moulded in contact therewith.

As a variant, not shown, the connection bridges 72 may be set back with respect to the grid 19, the latter not being in contact with the connection bridges 72 and coming to press against the annular central region 75.

The connection bridges 72 then make it possible to connect the annular central region 75 to the support part 16.

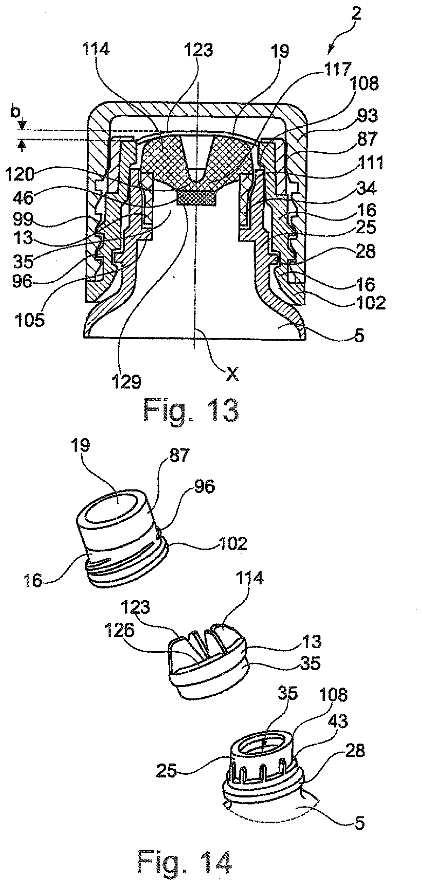

The embodiment shown in Figures 13 and 14 differs from the preceding embodiment in the shape of the insert 13 and of the support element 22.

The support element 22 comprises a plurality of partitions in the form of fins 114 which are connected at their lower end 117 to a central portion 120 and are in contact with the grid 19 at their upper edge 123.

Two adjacent fins 114 define, between them, a passage 126 for product from the receptacle 5 towards the grid 19, along the axis X of the applicator head 10.

The central portion 120 preferably forms a plate 129 defining a jet break 46. The jet break 46 is preferably arranged within the passage 35 formed by the sealing skirt 34.

The fins 114 are preferably moulded with the insert 13, separately from the grid 19.

The insert 13 may be force-fitted into the neck 25 of the receptacle 5 by means of its sealing skirt 34.

The receptacle may comprise a stop 105 limiting how far the insert 13 can be pushed into the neck 25 and/or, as a variant, the insert 13 may widen above the sealing skirt 34 so as to abut against the upper edge 108 of the neck 25 or so as to lodge in a widened portion 111 of the inside of the neck 25.

The support part 16 is fixed to the receptacle 5 by any suitable means, in particular by snap-fitting, and the grid 19 comes into contact with the upper edge 123 of the fins 114.

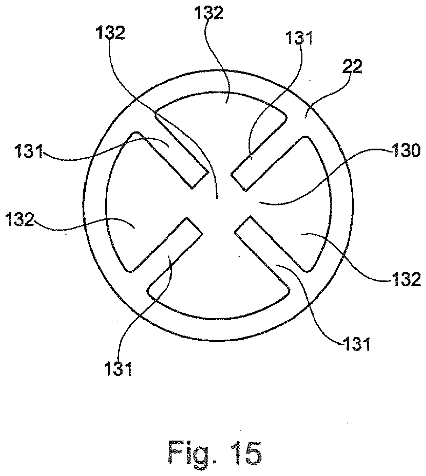

As a variant, shown in Figure 15, the support element 22 may comprise a product outlet forming a single opening 130 which is separated, by a plurality of partitions 131, into a plurality of distribution regions 132. The partitions 131 are designed to support the grid 19.

Of course, the invention is not limited to the examples illustrated.

It is for example possible to use other materials to produce the applicator head.

The applicator head can be attached to the receptacle and/or the support part can be attached to the insert by other means, in particularly by screwing or force-fitting.

The expression "having a" should be understood as being synonymous with "having at least one".

## Claims

1. Device (2) for packaging and applying a cosmetic or dermatological product, comprising:
- a receptacle (5) containing said cosmetic or dermatological product,
- an applicator head (10) which is supplied with product by the receptacle (5), the applicator head (10) comprising:
∘ a grid (19) defining an application surface (19a) for applying the product onto a surface to be treated,
∘ a partitioned support element (22) for the grid (19), against which the grid (19) can rest at least during application, having at least one partition (72, 114) between two regions (78, 126) where the product can pass through the support element.

2. Device according to Claim 1, the grid (19) being a woven or non-woven textile or an injected or extruded net.

3. Device according to Claim 1 or 2, the grid (19) being a textile and the textile being a woven, or being perforated, or being non-perforated.

4. Device according to any one of the preceding claims, the grid (19) having an application surface (19a) which is domed towards the surface to be treated.

5. Device according to any one of the preceding claims, the support element (22) being in contact with the grid (19).

6. Device according to any one of the preceding claims, the support element (22) comprising a plurality of partitions, the partitions being connection bridges (72) connected to a central region (75), the connection bridges (72) defining, between them, openings (78) the central region (75) being open-worked, or the partitions being fins (114) connected to a central region (129), the fins (114) defining, between them, axial passages (126) for the product.

7. Device according to any one of the preceding claims, the applicator head (10) comprising a sealing skirt (34) which engages in the receptacle (5) and/or comprising a jet break (46) making it possible to improve the distribution of the product over the entire grid (19), and/or comprising an insert (13) which is attached, in particular snap-fitted, onto the receptacle (5), or which is forced into the receptacle (5), and a support part (16) which bears the grid (19) and is attached to the insert (13) or the receptacle (5), in particular snap-fitted onto the insert (13) or the receptacle (5).

8. Device according to any one of the preceding claims, the applicator head (10) being at least partly attached to the receptacle (5), in particular secured by snap-fitting onto the receptacle (5).

9. Device according to any one of the preceding claims, the applicator head (10) and the receptacle (5) comprising cooperating anti-rotation reliefs, in particular cooperating projecting elements (43) and recesses (40), or the support part (16) and the insert (13) or the receptacle (5) comprising cooperating anti-rotation reliefs, in particular cooperating splines (60, 63).

10. Device according to any one of the preceding claims, the grid (19) being made of a thermoplastic material, in particular polyamide 6.6, PP, PA or PET.

11. Device according to any one of the preceding claims, the grid (19) comprising a plurality of threads (81) of diameter *d* between 2 µm and 1000 µm, preferably between 50 µm and 250 µm, the grid (19) comprising preferably between 20 threads/cm and 70 threads/cm, preferably between 28 threads/cm and 35 threads/cm..

12. Device according to any one of the preceding claims, the grid (19) being formed from a woven or a net, the mesh of the grid (19) having openings of cross section S between 0.01 mm² and 1 mm², preferably between 0.02 mm² and 0.01 mm².

13. Device according to any one of the preceding claims, the applicator head (10), in particular the support part (16), comprising a portion which is overmoulded onto the grid (19), and/or comprising a portion which is fixed, in particular overmoulded, onto a support ring (87) which is itself overmoulded onto the grid (19).

14. Device according to any one of the preceding claims, the receptacle (5) having a variable internal volume, the supply of product from the receptacle (5) to the applicator head (10) being brought about by pressure on a wall of the receptacle (5).

## Patentansprüche

1. Vorrichtung (2) zum Verpacken und Auftragen eines kosmetischen oder dermatologischen Produkts, die Folgendes umfasst:
- einen Behälter (5), der das kosmetische oder dermatologische Produktenthält,
- einen Applikatorkopf (10), der mit dem Produkt durch den Behälter (5) beliefert wird, wobei der Applikatorkopf (10) Folgendes umfasst:
∘ ein Gitter (19), das eine Auftragsfläche (19a) zum Auftragen des Produkts auf eine zu behandelnde Oberfläche definiert,
∘ ein unterteiltes Trägerelement (22) für das Gitter (19), an dem das Gitter (19) mindestens während des Auftragens anliegen kann, das mindestens eine Abtrennung (72, 114) zwischen zwei Bereichen (78, 126) aufweist, wo das Produkt das Trägerelement durchdringen kann.

2. Vorrichtung nach Anspruch 1, wobei das Gitter (19) ein gewebter oder nichtgewebter Stoff oder ein gespritztes oder extrudiertes Netz ist.

3. Vorrichtung nach Anspruch 1 oder 2, wobei das Gitter (19) ein Stoff ist und der Stoff ein Gewebe ist, perforiert ist oder unperforiert ist.

4. Vorrichtung nach einem der vorhergehenden Ansprüche, wobei das Gitter (19) eine Auftragsfläche (19a) besitzt, die zu der zu behandelnden Oberfläche gewölbt ist.

5. Vorrichtung nach einem der vorhergehenden Ansprüche, wobei das Trägerelement (22) in Kontakt mit dem Gitter (19) ist.

6. Vorrichtung nach einem der vorhergehenden Ansprüche, wobei das Trägerelement (22) mehrere Abtrennungen umfasst, wobei die Abtrennungen mit einem zentralen Bereich (75) verbundene Verbindungsbrücken (72) sind, die Verbindungsbrücken (72) zwischen sich Öffnungen (78) definieren und der zentrale Bereich (75) durchbrochen ist, oder die Abtrennungen mit einem zentralen Bereich (129) verbundene Lamellen (114) sind und die Lamellen (114) zwischen sich axiale Durchgänge (126) für das Produkt definieren.

7. Vorrichtung nach einem der vorhergehenden Ansprüche, wobei der Applikatorkopf (10) eine Dichtungsschürze (34) umfasst, die in den Behälter (5) eingreift, und/oder einen Strahlunterbrecher (46) umfasst, der es ermöglicht, die Verteilung des Produkts über das gesamte Gitter (19) zu verbessern, und/oder Folgendes umfasst: einen Einsatz (13), der insbesondere durch Einrasten auf dem Behälter (5) befestigt ist oder der in den Behälter (5) gedrückt ist, und ein Trägerelement (16), das das Gitter (19) trägt und insbesondere durch Einrasten auf dem Einsatz (13) oder dem Behälter (5) mit dem Einsatz (13) bzw. dem Behälter (5) verbunden ist.

8. Vorrichtung nach einem der vorhergehenden Ansprüche, wobei der Applikatorkopf (10) mindestens teilweise, insbesondere gesichert durch Einrasten auf dem Behälter (5), an dem Behälter (5) befestigt ist.

9. Vorrichtung nach einem der vorhergehenden Ansprüche, wobei der Applikatorkopf (10) und der Behälter (5) zusammenwirkende Drehblockierungsreliefs, insbesondere zusammenwirkende vorstehende Elemente (43) und Aussparungen (40), umfassen oder das Trägerelement (16) und der Einsatz (13) oder der Behälter (5) zusammenwirkende Drehblockierungsreliefs, insbesondere zusammenwirkende Kerbverzahnungen (60, 63), umfassen.

10. Vorrichtung nach einem der vorhergehenden Ansprüche, wobei das Gitter (19) aus einem thermoplastischen Material, insbesondere Polyamid 6,6, PP, PA oder PET, hergestellt ist.

11. Vorrichtung nach einem der vorhergehenden Ansprüche, wobei das Gitter (19) mehrere Fäden (81) des Durchmessers d im Bereich von 2 µm bis 1000 µm, vorzugsweise im Bereich von 50 µm bis 250 µm, umfasst, wobei das Gitter (19) vorzugsweise zwischen 20 Fäden/cm und 70 Fäden/cm, vorzugsweise zwischen 28 Fäden/cm und 35 Fäden/cm, umfasst.

12. Vorrichtung nach einem der vorhergehenden Ansprüche, wobei das Gitter (19) aus einem Gewebe oder einem Netz gebildet ist und die Maschenweite des Gitters (19) Öffnungen des Querschnitts S im Bereich von 0,01 mm² bis 1 mm², vorzugsweise im Bereich von 0,02 mm² bis 0,01 mm², aufweist.

13. Vorrichtung nach einem der vorhergehenden Ansprüche, wobei der Applikatorkopf (10), insbesondere das Trägerelement (16), einen Teil umfasst, der auf das Gitter (19) spritzgegossen ist, und/oder einen Teil umfasst, der an einem Trägerring (87), der selbst auf das Gitter (19) spritzgegossen ist, befestigt ist, insbesondere durch Spritzgießen.

14. Vorrichtung nach einem der vorhergehenden Ansprüche, wobei der Behälter (5) ein variables Innenvolumen besitzt und die Produktzufuhr von dem Behälter (5) zu dem Applikatorkopf (10) durch Druck auf eine Wand des Behälters (5) herbeigeführt wird.

## Revendications

1. Dispositif (2) de conditionnement et d'application d'un produit cosmétique ou dermatologique, comportant :
un récipient (5) contenant ledit produit cosmétique ou dermatologique,
une tête applicatrice (10) alimentée en produit par le récipient (5), la tête d'application (10) comportant :
une grille (19) définissant une surface d'application (19a) pour appliquer le produit sur une surface à traiter,
un élément cloisonné de soutien (22) de la grille (19), contre lequel la grille (19) peut reposer au moins lors de l'application, présentant au moins une cloison (72, 114) entre deux zones (78, 126) où le produit peut traverser l'élément de soutien.

2. Dispositif selon la revendication 1, la grille (19) étant un textile tissé ou non tissé ou un filet injecté ou extrudé.

3. Dispositif selon la revendication 1 ou 2, la grille (19) étant un textile et le textile étant un tissé, ou étant perforé, ou étant non perforé.

4. Dispositif selon l'une quelconque des revendications précédentes, la grille (19) présentant une surface d'application (19a) bombée vers la surface à traiter.

5. Dispositif selon l'une quelconque des revendications précédentes, l'élément de soutien (22) étant en contact avec la grille (19).

6. Dispositif selon l'une quelconque des revendications précédentes, l'élément de soutien (22) comprenant une pluralité de cloisons, les cloisons étant des ponts de liaison (72) reliés à une région centrale (75), les ponts de liaison (72) définissant, entre eux, des ouvertures (78), la région centrale (75) étant ajourée ou les cloisons étant des ailettes (114) reliées à une région centrale (129), les ailettes (114) définissant, entre elles, des passages axiaux (126) pour le produit.

7. Dispositif selon l'une quelconque des revendications précédentes, la tête applicatrice (10) comportant une jupe d'étanchéité (34) s'engageant dans le récipient (5) et/ou comportant un brise-jet (46) permettant d'améliorer la répartition du produit sur l'ensemble de la grille (19), et/ou comprenant un insert (13) rapporté, en particulier encliqueté, sur le récipient (5), ou rentré en force dans le récipient (5), et une pièce de support (16), portant la grille (19) et rapporté sur l'insert (13) ou le récipient (5), en particulier encliquetée sur l'insert (13) ou le récipient (5).

8. Dispositif selon l'une quelconque des revendications précédentes, la tête applicatrice (10) étant au moins en partie rapporté sur le récipient (5), en particulier fixée par encliquetage sur le récipient (5).

9. Dispositif selon l'une quelconque des revendications précédentes, la tête d'application (10) et le récipient (5) comportant des reliefs anti-rotation coopérants, en particulier des éléments en saillie (43) et des évidements (40) coopérants, ou la pièce de support (16) et l'insert (13) ou le récipient (5) comprenant des reliefs anti-rotation coopérants, en particulier des cannelures (60, 63) coopérantes.

10. Dispositif selon l'une quelconque des revendications précédentes, la grille (19) étant en matière thermoplastique, en particulier en polyamide 6.6, PP, PA ou PET.

11. Dispositif selon l'une quelconque des revendications précédentes, la grille (19) comportant une pluralité de fils (81) de diamètre d compris entre 2 µm et 100 µm, de préférence_compris entre 50 µm et 250 µm, la grille (19) comportant de préférence entre 20 fils/cm et 70 fils/cm, de préférence entre 28 fils/cm et 35 fils/cm.

12. Dispositif selon l'une quelconque des revendications précédentes, la grille (19) étant formée à partir d'un tissé ou d'un filet, la maille de la grille (19) ayant chacune une ouverture de section transversale S comprise entre 0,01 mm² et 1 mm², de préférence comprise entre 0,02 mm² et 0,01 mm².

13. Dispositif selon l'une quelconque des revendications précédentes, la tête applicatrice (10), en particulier la pièce de support (16), comportant une portion surmoulée sur la grille (19) et/ou comportant une portion fixée, en particulier surmoulée, sur une bague de support (87) elle-même surmoulée sur la grille (19).

14. Dispositif selon l'une quelconque des revendications précédentes, le récipient (5) étant à volume intérieur variable, l'alimentation en produit du récipient (5) à la tête applicatrice (10) se faisant par pression sur une paroi du récipient (5).
